# EUROPEAN PATENT SPECIFICATION

(11) **EP 1 289 520 B1**
(45) Date of publication and mention of the grant of the patent: **17.08.2005**
(21) Application number: 01943711.0
(22) Date of filing: 06.06.2001
(51) Int. Cl.: A61K 31/341, A61P 25/24

(54) **PHARMACEUTICAL COMPOSITIONS FOR THE TREATMENT OF DEPRESSION COMPRISING FAMOTIDINE**
PHARMAZEUTISCHE ZUSAMMENSETZUNG ENTHALTEND FAMOTIDINE FÜR DIE BEHANDLUNG VON DEPRESSIONEN
COMPOSITIONS PHARMACEUTIQUE POUR LE TRAITEMENT DE LA DEPRESSION OU DES SYMPTOMES DE LA DEPRESSION

(30) Priority: 06.06.2000 HU 0002154 P
(43) Date of publication of application: 12.03.2003
(73) Proprietor: RICHTER GEDEON VEGYESZETI GYAR R.T., 1103 Budapest X (HU)
(72) Inventor: RACZ, Anna, H-1021 Budapest (HU); KOVACS, Péter, H-4028 Debrecen (HU); VARGA, Csilla, H-4400 Nyiregyháza (HU)
(74) Representative: Bösl, Raphael, Dr. rer. nat., Dipl.-Chem.
(86) International application number: PCT/HU2001/000065
(87) International publication number: WO 2001/093863

(56) References cited:
- WO-A-95/01780
- WO-A-95/01784
- US-A- 5 352 688
- MOLINARI S P ET AL: "THE USE OF FAMOTIDINE IN THE TREATMENT OF PARKINSON'S DISEASE: A PILOT STUDY" JOURNAL OF NEURAL TRANSMISSION, SPRINGER VERLAG, VIENNA, AT, vol. 9, no. 2/3, 23 August 1995 (1995-08-23), pages 243-247, XP000673243 ISSN: 0300-9564
- DANNON P N ET AL: "Famotidine: A supplemental drug for the treatment of schizophrenia." EUROPEAN PSYCHIATRY, vol. 12, no. 5, 1997, pages 263-264, XP002188477 ISSN: 0924-9338

## Description

The present invention relates to the administration of pharmaceutical preparations containing the active ingredient famotidine, in the treatment of depression or symptoms suggesting depression.

Recently, depression can be regarded as an epidemic affecting the whole population all over the world. According to a national representative assay in Hungary (Kopp M et al, Lege Artis Med. 7(3): 136-144, 1997), symptoms of depression occurred in 24.3% of the population in 1988, while this number turned out to be 30.6% by 1995. The information that in 1988 only 2.9% of the population was affected by depression of the severity that required therapy, while this figure was already 7.1% in 1995, is of even higher value. These data clearly demonstrate that the sudden increase, especially in the number of the patients requiring therapy, brought into focus the need of medicinal therapy and the launching of more specific medicines.

It is also a widely known fact that the group of patients suffering in gastrointestinal diseases is much more prone to hazards relating to depression, in other words they often develop different forms of depression or symptoms suggesting depression (for example functional dyspepsia). These may counteract on the underlying disease, often impeding or preventing recovery. Therefore it could be desirable, if pharmaceutical preparations developed for gastrointestinal diseases also had a positive effect on complaints relating to depression, but at least they should not worsen the clinical case.

Unfortunately, the applied preparations show a rather heterogeneous picture. Several publications - e.g. Hassan and Saieed (Eur. J. Pharm. Sci., 6, Suppl. 1, S88, 1998) - have dealt with the psychiatric effects of H₂-receptor antagonistsie with a high sales record. The firstly developed member of the group, cimetidine, was found to have a marked depressant effect (see e.g. Mangla J.C., Clin.Res. 33., No. 2, Pt. 1, 323 A, 1985 or Rush P.J., Am.J.Med.Sci. 286, No.3, 31-34, 1983).

One of the most successful members of the group of H₂-receptor antagonists is ranitidine, and the original medicine containing this active ingredient is presently marketed by the name of Zantac® (Glaxo Wellcome). We have to anticipate a similar effect in the case of ranitidine as in the case of cimetidine. Billings and Stein (Am. J. Psychiatry, 143, No.7, 915-15, 1986) have reported that the development of depression could be unquestionably associated with ranitidine-therapy in several cases. As a matter of fact, the manifest symptoms disappeared after terminating the administration of rantidine. Pharmaceutical guides, such as the Physicians' Desk Reference, also quote depression among the adverse reactions that are related to the administration of ranitidine (Physicians' Desk Reference, 1999., page 1309). Nevertheless, we also have to mention that Robins et al. (Postgrad. Med. J., 60, No. 703, 353-55, 1884) have come to the conclusion that.the mood of patients receiving ranitidine improved at a higher rate than individuals in the control-group receiving placebo. The reason behind the positive mood change was probably that the sensation of pain due to the ulcer diminished as a result of the applied therapy, and obviously, as a consequence, the mood of the patient has also improved in parallel. Famotidine, the molecule launched after ranitidine, is the active ingredient of several pharmaceutical preparations (Pepcid®-Merk, Quamatel®-Richter Gedeon, Gaster® Yamanouchi). On the basis of the professional literature, famotidine has a similar gastrointestinal efficacy as ranitidine, but associated with less and milder adverse reactions. Pharmaceutical guides do mention however depression as one of the adverse reactions related to the administration of famotidine (Physicians' Desk Reference, 1999, page 1854.) The majority of publications made in psychiatry and worthy of mentioning are case studies, which report that famotidine is effective in the management of schizophrenia.

Therefore it appears that famotidine has a more beneficial effect in the treatment of gastrointestinal complaints due to the milder and less frequent occurrence of adverse reactions. We have set out the objective to investigate the efficacy of famotidine and ranitidine individually, and also in comparison with one another, furthermore to record the adverse reactions potentially leading to depression.

During the study we surprisingly found that while practically displaying the same gastrointestinal efficacy, famotidine resulted in a significant reduction both in the values of depression and anxiety in the study population. The positive change was significant in the case of the symptoms suggesting depression, not only in comparison with the ranitidine-group, but also in comparison with the baseline values, i.e. famotidine had a significantly detectable antidepressant effect.

On the basis of the above mentioned information, the invention relates to the human therapeutic application of famotidine and its salts in the treatment of depression or symptoms suggesting depression, including somatic depression, unipolar depression, functional illnesses of psychic origin, atypical depression, dysthymia, bipolar affective disorder, seasonal depression and persistent mood disorder.

During the multicenter, controlled clinical study, patients received medicinal therapy for a period of four weeks, i.e. they either received Quamatel® (famotidine) tablets in a daily dose of 2x20 mg, or Zantac® (ranitidine) tablets in a daily dose of 2x150 mg. The number of cases investigated was 119, which assured that the obtained data reach the level of statistical significance. The psychiatrist - who had no information regarding the treatment group the patient was assigned to - had performed the evaluation according to the Hamilton 24 (HAMD) depression scale, the Montgomery-Asberg (MADRS) depression scale or the Hamilton (HAMA) anxiety rating scale. During the statistical evaluation a probability level of p≤0.05 was regarded as significant.

Following therapy there was a significant reduction in the overall scores observed in both treatment groups when assessed by the HAMD, the MADRS and the Hamilton anxiety scales (p<0001). When comparing the two treatment groups according to the HAMD scale, the extent of improvement proved to be statistically higher in the Quamatel® treatment-group than in the Zantac® treatment-group (p=0.048). Although, when comparing the two treatment groups by the MADRS and HAMA scales the numerical difference obtained was not statistically significant (p=0.14 and p=0.19, respectively), nevertheless, the Quamatel® treatment-group demonstrated better results in this case as well. These results were only supported by the overall subjective assessment parameters: the patients' opinion of the mood improvement (p=0.001), and the psychiatrist's evaluation regarding the changes in the symptoms suggesting depression (p=0.005) was significantly better in the Quamatel®-group than in the Zantac®-group in the statistical sense.

This significant difference had only been observable regarding the symptoms suggesting depression, and it did not apply to the improvement of the general condition assessed by the patients themselves (p=0.93), to the change in the extent of anxiety assessed by the psychiatrist (p=0.011), and to the improvement in the gastroenterological condition of the patient assessed by the gastroenterologist (p=0.035). All three psychiatric scales and all global assessment parameters demonstrated significant improvement in the case of both therapies.

The improvement of the mood and the depressive factors had shown a statistically significant difference in favor of the Quamatel® treatment-group both according to the patients' and the psychiatrist's opinion (scoring assessment). On the basis of the results it can be concluded that famotidine has a specific depression-reducing effect, which is a surprising, new finding taking into consideration the results of the professional literature available up to date. This surpasses the similar effect of ranitidine, which - being in harmony with the results of the professional literature - does not result from the antidepressant effect of ranitidine, but from the diminishment of the gastrointestinal complaints due to the applied therapy. In the case of famotidine though, the improvement in the psychic condition of the patients greatly surpasses the improvement of the psychic condition attributable to the improvement in the gastroenterological condition, thus it becomes evident that famotidine has a depression-diminishing effect of its own.

### Detailed data of the study

### Methods

Multicenter, randomized, controlled study; the study can be regarded blind with respect to the psychiatrist's assessment of the results.

### Number of patients

Planned: 120
Analyzed: 119
Gender: females and males (63:56)
Age distribution: 16-76 years

### Inclusion criteria

Therapy justified by dyspepsia, indicated by a gastroenterologist (ulcer, reflux esophagitis, gastric hyperacidity).

The patient's signature on the consent form.

### Exclusion criteria

Severe hepatic insufficiency, inadequate cooperation, pregnancy, lactation, alcoholism, influenza, known hypersensitivity to ranitidine or famotidine, ulcer necessitating H.pylori eradication, pneumonia, meningitis, diabetes mellitus, anemia, hypertension, neuroleptics, acute hepatitis, epilepsy, endocrinological diseases, psychiatric treatment due to depression, antidepressant pharmaceutical therapy, cerebrovascular diseases, tumors, Parkinson's-disease, cerebral commotion, dementia, the use of H₂-receptor blockers within the past month.

### Duration of therapy

4-week treatment period with the administration of the test- or reference medication.

### The course of the study

Prior to treatment: On the occasion of the 1^{st} visit (week -1-0) the recording of the medical history, physical examination, endoscopy (optional), the signing of the consent form, then the assessment of the extent of anxiety during the psychiatric examination, by using the Hamilton 24 (HAMD) and the MADRS (Montgomery Asberg) depression scales as well as the Hamilton Anxiety Rating Scale (HAMA).

Treatment period: This is followed by a four-week (week 0-4) H₂-blocker therapy performed on the basis of the former randomization applying either Quamatel®- or Zantac®-therapy. During randomization they have also taken the female-male ratio into consideration. Following the four-week treatment they have performed a gastroenterological control examination at visit 2 of week 4, which was followed by the psychiatric control examination to determine the extent of depression using the HAMD and the MADRS depression scales, as well as the HAMA anxiety rating scale.

### Efficacy assessment criteria (endpoint variables)

The depression altering effects of H₂-receptor blockers have been assessed on the basis of the overall scores obtained by the HAMD and the MADRS depression scales prior to treatment and following the 4-week therapy.
- Hamilton 24 (HAMD) depression scale: 24 parameters, 0-4 point scale
- MADRS depression scale: 10 parameters, 0-6 point scale
- Hamilton anxiety rating scale (HAMA): 15 parameters, 0-3 point scale
- The psychiatrist assessed the changes in the patients' symptoms of depression and anxiety by using a score-range of 0-5.

### Safety assessment criteria (endpoint variables): Statistical methods

The statistical evaluation of the study results was performed by using the software called SAS for Windows 6.12 (SAS Institute Inc., Cary, NC, U.S.A) on a computer.

The describing statistics (median, quartiles, average, dispersion, case-number, minimum and maximum values) were given for all numerical data variables (age, body weight, height, overall scores for HAMD and MADRS, as well as HAMA). To assess the real-time changes in the overall scores for HAMD and MADRS, as well as HAMA between the two treatment-groups, repeated ANOVA procedures (SAS, GLM method) had been applied to the values formerly transformed logarithmically. The global parameters (the patient's opinion of the changes in the mood and the general condition, the physician's opinion of the general condition of the patient, and the psychiatrist's opinion of the changes in depression and anxiety) were assessed by applying the median-test (SAS NPAR1WAY method).

Probability levels of<0.05 were considered significant during the statistical evaluations.

### Results, efficacy outcomes

### Quamatel®-group

| Total Scores of the Psychiatric Tests | | | | | | |
|---|---|---|---|---|---|---|
| | HAM Depression | | MADRS | | HAMA Anxiety | |
| | Before Therapy | After Therapy | Before Therapy | After Therapy | Before Therapy | After Therapy |
| Case number | 60 | 60 | 60 | 60 | 60 | 60 |
| Median | 9 | 5 | 8 | 4 | 8 | 5.5 |
| Interquartile range | 6-12 | 3-9 | 4-11 | 2-8 | 5-11 | 3,5-8,5 |
| Average | 9.65 | 6.22 | 8.78 | 5.72 | 8.37 | 6.43 |
| Std.Deviatio | 5.77 | 4.89 | 6.64 | 4.84 | 4.34 | 4.74 |
| Min-Max | 1-27 | 0-24 | 0-33 | 0-28 | 2-23 | 1-23 |

### Zantac® group

| Total Scores of the Psychiatric Tests | | | | | | |
|---|---|---|---|---|---|---|
| | HAM Depression | | MADRS | | HAMA Anxiety | |
| | Before Therapy | After Therapy | Before Therapy | After Therapy | Before Therapy | After Therapy |
| Case number | 59 | 59 | 59 | 59 | 59 | 59 |
| Median | 10 | 7 | 8 | 6 | 9 | 6 |
| Interquartile range | 7-13 | 4-11 | 4-16 | 2-10 | 5-14 | 4-10 |
| Average | 11.18 | 8.68 | 10.73 | 10.73 | 9.8 | 7.92 |
| Std.Deviation | 6.31 | 6.64 | 8.17 | 8.17 | 6.05 | 5.78 |
| Min-Max | 1-28 | 1-32 | 0-38 | 0-38 | 1-27 | 1-25 |

### Total

| Total Scores of the Psychiatric Tests | | | | | | |
|---|---|---|---|---|---|---|
| | HAM Depression | | MADRS | | HAMA Anxiety | |
| | Before Therapy | After Therapy | Before Therapy | After Therapy | Before Therapy | After Therapy |
| Case number | 119 | 119 | 119 | 119 | 119 | 119 |
| Median | 10 | 6 | 8 | 4 | 8 | 6 |
| Interquartile range | 6-13 | 3-10 | 4-14 | 2-10 | 5-12 | 4-9 |
| Average | 10.36 | 7.44 | 9.75 | 7.26 | 9.08 | 7.17 |
| Deviation | 6.06 | 5.93 | 7.47 | 7.08 | 5.28 | 5.33 |
| Min-Max | 1-28 | 0-32 | 0-38 | 0-38 | 1-27 | 1-25 |

### Quamatel®

| | According to the patient's opinion, did the mood improve in parallel with the therapy? | Did the symptoms of depression change in parallel with the therapy according to the psychiatrist's opinion? |
|---|---|---|
| Average | 3.51 | 3.32 |
| Deviation | 0.63 | 0.60 |
| Minimum | 2 | 2 |
| Maximum | 5 | 5 |
| Median | 3 | 3 |
| Interquartile range | 3-3.4 | 3-4 |

### Zantac®

| | According to the patient's opinion, did the mood improve in parallel with the therapy? | Did the symptoms of depression change in parallel with the therapy according to the psychiatrist's opinion? |
|---|---|---|
| Average | 3.14 | 2.93 |
| Deviation | 0.68 | 0.69 |
| Minimum | 1 | 1 |
| Maximum | 4 | 4 |
| Median | 3 | 3 |
| Interquartile range | 3-4 | 3-3 |

The studies can be conducted with the pharmaceutical composition prepared according to the example introduced below, without restricting the scope of the invention to this.

### The preparing of tablets with famotidine-content

Tablets are prepared from the active ingredient famotidine by the method of direct pressing.

| Weights : | |
|---|---|
| famotidine | 20.0g |
| magnesium stearate | 2.0g |
| talc | 4.0g |
| polyvinyl-pirrolidon | 6.5g |
| starch | 37.0g |
| microcrystalline cellulose | 37.0g |
| lactose | 43.5g |

The listed amount of ingredients are homogenized in the form of a powder (gross weight: 150g), then it is filled into a tablet press to give 1000 tablets.
The individual weight of the tablets is 150 mg, and the active ingredient content is 20 mg of famotidine.

## Claims

1. Use of famotidine for the preparation of a pharmaceutical composition for the treatment of depression, depressive disorders and symptoms suggesting depression.

2. Use of claim 1 wherein the depression, depressive disorders and symptoms suggesting depression are represented by somatic depression, unipolar depression, atypical depression, dysthymia, bipolar affective disorders, seasonal de pressions and persistent mood disorder.

3. Use of claim 1 wherein the pharmaceutical composition is **characterized by** mixing 0.1-99.9 weight ratio of famotidine or its therapeutically acceptable salt with 0.1-99.9 weight ratio of one ore more conventional pharmaceutical excipients, converting it into a pharmaceutical composition by methods known from prior art.

## Patentansprüche

1. Verwendung von Famotidin zur Herstellung eines Arzneimittels zur Behandlung von Depression, depressiven Störungen und auf Depression hinweisenden Symptomen.

2. Verwendung nach Anspruch 1, wobei die Depression, depressiven Störungen und die auf Depression hinweisenden Symptome durch somatische Depression, unipolare Depression, atypische Depression, Dysthymie, manisch-depressive affektive Psychosen, saisonale Depressionen und anhaltende Gemütskrankheit dargestellt sind.

3. Verwendung nach Anspruch 1, wobei das Arzneimittel **gekennzeichnet ist durch** Mischen von 0,1-99,9 Gew.-% Famotidin oder seines therapeutisch verträglichen Salzes mit 0,1-99,9 Gew.-% eines oder mehreren herkömmlichen pharmazeutischen Exzipienten, Umwandeln dessen in ein Arzneimittel **durch** auf dem Stand der Technik bekannte Verfahren.

## Revendications

1. Utilisation de la famotidine pour la préparation d'une composition pharmaceutique pour le traitement de la dépression, des désordres dépressifs et des symptômes suggérant la dépression.

2. Utilisation selon la revendication 1, où la dépression, les désordres dépressifs et les symptômes suggérant la dépression sont représentés par une dépression somatique, une dépression unipolaire, une dépression atypique, une dysthymie, des désordres affectifs bipolaires, des dépressions saisonnières et un désordre persistant de l'humeur.

3. Utilisation selon la revendication 1, où la composition pharmaceutique est **caractérisée par** le mélange d'un ratio pondéral de 0,1-99,9 de famotidine ou de son sel thérapeutiquement acceptable avec un ratio pondéral de 0,1-99,9 d'un ou plusieurs excipients pharmaceutiques conventionnels, sa conversion en une composition pharmaceutique par des procédés connus de l'art antérieur.
